**Europäisches Patentamt**

**European Patent Office**

**Office européen des brevets**

(19)

(11) Veröffentlichungsnummer: **0 301 373 B1**

(12) **EUROPÄISCHE PATENTSCHRIFT**

(45) Veröffentlichungstag der Patentschrift: **21.11.91**

(21) Anmeldenummer: **88111574.5**

(22) Anmeldetag: **19.07.88**

(51) Int. Cl.⁵: **A61K 31/505**, //(A61K31/505, 31:275)

(54) **Erzeugnisse, enthaltend Gallopamil und Prazosin.**

(30) Priorität: **25.07.87 DE 3724644**

(43) Veröffentlichungstag der Anmeldung:
**01.02.89 Patentblatt 89/05**

(45) Bekanntmachung des Hinweises auf die
Patenterteilung:
**21.11.91 Patentblatt 91/47**

(84) Benannte Vertragsstaaten:
**AT BE CH DE FR GB IT LI NL**

(56) Entgegenhaltungen:
**EP-A- 0 189 336**

**DIALOG, Nr.04176814, medline 66-89/August;
H. GLOSSMANN et al.:"Calcium-and
potassium-channel blockers interact with
alpha-adrenoceptors", & MOL. CELL. ENDO-
CRINOL, Sep. 1980, 19(3) p243-51**

**Comprehensive Medicinal Chemistry, Band
6, S. 553**

(73) Patentinhaber: **KNOLL AG
Knollstrasse
W-6700 Ludwigshafen(DE)**

(72) Erfinder: **Tetzner, Christine, Dr.
Bleichstrasse 23
W-6700 Ludwigshafen(DE)**

(74) Vertreter: **Höller, Klaus, Dr. et al
BASF Aktiengesellschaft Carl-Bosch-Strasse
38
W-6700 Ludwigshafen(DE)**

## Beschreibung

Es ist bereits bekannt, daß Gallopamil und Prazosin blutdrucksenkende Eigenschaften besitzen (Drug Research 20, 799-802 (1970), medwelt 35, 1534 (1984), Münch. med. Wschr. 127, 379 (1985)). Es ist weiter bekannt, daß eine Kombination von Verapamil und Prazosin überadditive Effekte besitzt und in Depotform gut verträglich ist (Clin. Pharmacol. Ther. 36, 716 (1984); Third European Meeting on Hypertension, Mailand Italien 14.-17.06.1987 (abstract 464), Second Annual Meeting of the American Society of Hypertension 16.-21.05.1987 (abstract A 282).

Es wurde nun gefunden, daß Kombinationen aus Gallopamil und Prazosin überadditive Eigenschaften besitzen.

Die vorliegende Erfindung betrifft Erzeugnisse, enthaltend Gallopamil und Prazosin als Kombinationspräparat zur gleichzeitigen Anwendung in der Therapie des Bluthochdruckes und von nicht-entzündlichen Erkrankungen des Herzens, wie z.B. Herzinsuffizienz.

Das Kombinationspräparat enthält das Gallopamil und das Prazosin vorzugsweise im Verhältnis von 400:1 bis 10:1, insbesondere 200:1 bis 50:1.

Da die beiden Substanzen basische Gruppen enthalten, können sie auch in Form ihrer Salze mit physiologisch verträglichen Säuren für das Kombinationspräparat verwendet werden. Als physiologisch verträgliche Säuren kommen vorzugsweise in Betracht: Salzsäure, Schwefelsäure, Phosphorsäure, Essigsäure, Zitronensäure, Malonsäure, Salicylsäure, Maleinsäure, Fumarsäure, Bernsteinsäure, Ascorbinsäure, Äpfelsäure, Methansulfonsäure, Milchsäure, Gluconsäure, Glucuronsäure, Amidosulfonsäure, Benzoesäure, Weinsäure.

Die neue Applikationsform wird oral verabfolgt. Hierzu eignen sich insbesondere Tabletten, Dragees und Kapseln.

Die tägliche Dosis beträgt vorzugsweise 100 mg Gallopamilhydrochlorid in Kombination mit 1 mg Prazosin.

Für die Herstellung der Depot- bzw. Retardformen können die üblichen Verfahren verwendet werden (vgl. H. Sucker et al.: Pharmazeutische Technologie, Thieme-Verlag, Stuttgart 1978). Die folgenden Beispiele schildern bevorzugte Ausführungsformen.

Beispiel 1

Es werden in üblicher Weise Tabletten folgender Zusammensetzung hergestellt:

| | |
|---|---|
| Gallopamil-hydrochlorid | 100,0 mg |
| Prazosin-hydrochlorid | 1,1 mg |
| Natriumalginat | 339,9 mg |
| Polyvinylpyrrolidon (M.G. 20.000) | 25,0 mg |
| Maisstärke | 40,0 mg |
| Magnesiumstearat | 4,0 mg |
| | 510,0 mg |

Die oben genannten Substanzen werden mit Ausnahme des Magnesiumstearats gemischt, mit Wasser durchfeuchtet, granuliert, getrocknet und gesiebt. Nach Zumischen des Magnesiumstearats werden Tabletten gepreßt. Die Tabletten werden anschließend mit einem magensaftlöslichen Filmlack auf Hydroxypropylbasis überzogen.

Beispiel 2

Die gemäß Beispiel 1 erhaltenen Tabletten werden mit einer Drageedecke überzogen, die aus 5 Teilen Rohrzucker, 2 Teilen Maisstärke, 2 Teilen Calciumcarbonat und 1 Teil Talkum besteht.

Beispiel 3

Es werden in üblicher Weise Pellets folgender Zusammensetzung hergestellt:

| | |
|---|---|
| Gallopamil-hydrochlorid | 100,0 mg |
| Prazosin-hydrochlorid | 2,0 mg |
| Zuckerpellets | 70,0 mg |
| Siliciumdioxid | 2,0 mg |
| Natriumdioctylsulfosuccinat | 2,0 mg |
| PVP (Kollidon® 30) | 15,0 mg |
| Mikrokristalline Cellulose | 20,0 mg |
| Polyethylenglykol 6000 | 3,0 mg |
| Polyethylenglykol 400 | 1,0 mg |
| Eudragit® RS | 20,0 mg |
| Talkum | 5,0 mg |
| | 240,0 mg |

Die Zuckerpellets werden in einem Dragierkessel vorgelegt. Anschließend wird eine Lösung von 5 % PVP in Wasser auf die Zuckerpellets gesprüht und eine pulvrige Mischung aus Gallopamil, Prazosin und Siliciumdioxid aufgetragen. Danach werden die so erhaltenen Pellets getrocknet. Der Vorgang wird wiederholt, bis die pulvrige Mischung und etwa 1/3 des PVP verbraucht sind. Danach wird das restliche PVP in Form einer 20 %igen Lösung portionsweise auf die Pellets aufgetragen und mit feinst vermahlener mikrokristalliner Cellulose besprüht. Nach jedem Auftrageschritt wird mit Warmluft getrocknet. Die so hergestellten Pellets werden anschließend mit einer Lösung von Dioctylsulfyl-Natriumsuccinat, Polyethylenglykol 6000 und 400 und Eudragit® RS filmbeschichtet.

Beispiel 4

30 Pellets Gallopamil-Retard und 30 Tabletten MINIPRESS®-retard werden jeweils paarweise in eine Durchdrückpackung gefüllt.

Die Gallopamil-Retard-Pellets werden analog Beispiel 3 jedoch ohne Zusatz von Prazosin hergestellt.

Klinische Studie

In einer therapeutischen Studie wurde antihypertensive Wirksamkeit und Verträglichkeit des Kombinationspräparates nach einmaliger oraler Gabe bei Patienten mit essentieller Hypertonie untersucht:

Nach einer 8-tägigen Vorbeobachtungsperiode, in der alle antihypertensiv wirksamen Medikamente abgesetzt wurden, erhielten die Patienten einmal 100 mg Gallopamil retard und nach einer 3-tägigen Auslaßperiode einmal 1 mg Prazosin retard. Nach einer weiteren 3-tägigen Auslaßperiode erhielten die Patienten einmal 100 mg Gallopamil retard zusammen mit 1 mg Prazosin retard.

Der systolische und diastolische Blutdruck wurden vor sowie 6, 8 und 12 Stunden nach oraler Gabe der Substanzen gemessen.

In die Prüfung wurden 4 männliche und 4 weibliche Patienten im Alter von durchschnittlich 54 Jahren mit essentieller Hypertonie einbezogen. In den Tabellen 1 und 2 sind die Mittelwerte des systolischen und diastolischen Blutdrucks vor sowie 6, 8 und 12 Stunden nach Gabe der Substanzen angegeben.

Wie Tabelle 1 zeigt, kam es im Vergleich zu den Ausgangswerten 6 Stunden nach Gabe von jeweils 100 mg Gallopamil retard sowie 1 mg Prazosin retard zu einer vergleichbaren Reduktion des systolischen Blutdrucks. Auch 8 Stunden nach Applikation war noch eine deutliche Blutdruckreduktion durch beide Einzelsubstanzen zu verzeichnen. 12 Stunden nach Gabe von Gallopamil retard war nur noch eine geringe systolische Blutdrucksenkung nachweisbar, während Prazosin retard zu diesem Zeitpunkt keine Wirkung mehr zeigte. Nach Gabe der Kombination beider Präparate war zu allen 3 Zeitpunkten ein gegenüber den Einzelsubstanzen überadditiver Effekt auf den systolischen Blutdruck zu verzeichnen, der 12 Stunden nach Applikation besonders deutlich war.

Besonders überraschend war, daß nach Gabe der Kombination von Gallopamil retard und Prazosin retard auch eine Verlängerung der antihypertensiven Wirkung beobachtet wurde. Die durch die Kombination erreichte Senkung des systolischen Blutdrucks war auch nach 24 Stunden noch erhalten, während der durch Gabe der Einzelsubstanzen erzielte Effekt auf den systolischen Blutdruck bereits 12 Stunden nach Einnahme deutlich nachgelassen hatte bzw. nicht mehr vorhanden war.

Bezüglich des mittleren diastolischen Blutdrucks (Tabelle 2) war 6, 8 und 12 Stunden nach oraler Applikation der Kombination von Gallopamil retard und Prazosin retard ein stärkerer Effekt als nach Gabe der Einzelsubstanzen zu verzeichnen.

Nebenwirkungen wurden bei den Untersuchungen mit der Kombination nicht beobachtet.

Tabelle 1

| | Gallopamil retard | | Prazosin retard | | Gallopamil retard + Prazosin retard | |
|---|---|---|---|---|---|---|
| | Mittelwert | Abnahme gegenüber dem Wert vor Einnahme | Mittelwert | Abnahme gegenüber dem Wert vor Einnahme | Mittelwert | Abnahme gegenüber dem Wert vor Einnahme |
| Stunden nach Einnahme | | | | | | |
| 0 | 179 | | 176 | | 176 | |
| 6 | 159 | -20 | 156 | -20 | 131 | -45 |
| 8 | 166 | -13 | 166 | -10 | 144 | -33 |
| 12 | 172 | - 7 | - | - | 146 | -30 |
| 24 | - | - | - | - | 150 | -26 |

Systolischer Blutdruck (mmHg)

Ein Bindestrich (-) bedeutet, daß keine klinisch relevante Wirkung gefunden wurde.

EP 0 301 373 B1

Tabelle 2

| Stunden nach Einnahme | Gallopamil retard | | Prazosin retard | | Gallopamil retard + Prazosin retard | |
|---|---|---|---|---|---|---|
| | Mittelwert | Abnahme gegenüber dem Wert vor Einnahme | Mittelwert | Abnahme gegenüber dem Wert vor Einnahme | Mittelwert | Abnahme gegenüber dem Wert vor Einnahme |
| 0 | 106 | | 107 | | 100 | |
| 6 | 96 | -10 | 93 | -14 | 81 | -19 |
| 8 | 95 | -11 | 95 | -12 | 82 | -18 |
| 12 | 99 | - 7 | 102 | - 5 | 84 | -16 |

Diastolischer Blutdruck (mmHg)

**Patentansprüche**

1. Erzeugnisse, enthaltend Gallopamil und Prazosin - jeweils in Depotform - als Kombinationspräparat zur gleichzeitigen Anwendung in der Therapie des Bluthochdrucks und von nicht-entzündlichen Erkrankungen des Herzens.

2. Erzeugnisse gemäß Anspruch 1, dadurch gekennzeichnet, daß sie Gallopamil und Prazosin im Verhältnis 400:1 bis 10:1 enthalten.

**Claims**

1. A product containing gallopamil and prazosin, each in a sustained-release form, as a combination preparation for simultaneous use in the therapy of high blood pressure and of noninflammatory cardiac disorders.

2. A product as claimed in claim 1, which contains gallopamil and prazosin in a ratio of from 400 : 1 to 10 : 1.

**Revendications**

1. Produits contenant du Gallopamil et du Prazosin - tous les deux sous une forme dépôt - en tant que composition combinée pour l'utilisation simultanée dans la thérapie de l'hypertension sanguine et des maladies cardiaques non inflammatoires.

2. Produits selon la revendication 1, caractérisés en ce qu'ils contiennent le Gallopamil et le Prazosin dans des proportions relatives de 400:1 à 10:1.